(19) Europäisches Patentamt
European Patent Office
Office européen des brevets

(11) EP 0 376 598 B1

(12) **EUROPEAN PATENT SPECIFICATION**

(45) Date of publication and mention of the grant of the patent:
**21.02.1996 Bulletin 1996/08**

(51) Int. Cl.[6]: **C07D 231/12**, C07D 231/16, A01N 43/56

(21) Application number: **89313324.9**

(22) Date of filing: **20.12.1989**

(54) **Pyrazole compounds, and their production and use**

Pyrazol-Verbindungen und ihre Herstellung und Verwendung

Dérivés de pyrazole et leur préparation et application

(84) Designated Contracting States:
**CH DE FR GB IT LI**

(30) Priority: **27.12.1988 JP 331076/88**

(43) Date of publication of application:
**04.07.1990 Bulletin 1990/27**

(73) Proprietor: **SUMITOMO CHEMICAL COMPANY, LIMITED**
**Chuo-ku Osaka 541 (JP)**

(72) Inventors:
- **Kisida, Hirosi**
  **Takarazuka-shi Hyogo-ken (JP)**
- **Nishida, Sumio**
  **Tokyo-to (JP)**
- **Shuto, Akira**
  **Takarazuka-shi Hyogo-ken (JP)**
- **Hatakoshi, Makoto**
  **Toyonaka-shi Osaka-fu (JP)**

(74) Representative: **Hardisty, David Robert et al**
**London EC4A IPQ (GB)**

(56) References cited:
**EP-A- 0 069 848** **EP-A- 0 262 344**
**WO-A-90/06678** **US-A- 3 190 888**

- **CHEMICAL ABSTRACTS, vol. 91, 1979, page 590, abstract no. 74523y, Columbus, Ohio, USA; H.J.M. DOU et al.: "Phase transfer catalysis in the azole series: N-alkylation of pyrazole", & AN. QUIM. 1978, 74(7-8), 1137-9**
- **CHEMICAL ABSTRACTS, vol. 111, 1989, pages 715-716, abstract no. 205323q, Columbus, Ohio, US; & JP-A-01 55 557**
- **CHEMICAL ABSTRACTS, vol. 101, 1984, page 47, abstract no. 8350p, Columbus,Ohio, US; & JP-A-59 15 469**
- **CHEMICAL ABSTRACTS, vol. 107, 1987, page 2, abstract no. 23735k, Columbus,Ohio, US; V.V. TSERUNYAN et al.: "Synthesis and polymerization of substituted 4-vinylpyrazoles", & ARM. KHIM. ZH. 1987, 40(1),48-54**
- **CHEMICAL ABSTRACTS, vol. 108, 1988, page 138, abstract no. 134239s, Columbus, Ohio, US; & JP-A-63 273 007**
- **CHEMICAL ABSTRACTS, vol. 109, 1988, page 257, abstract no. 50259f, Columbus, Ohio, USA; & JP-A-63 02 904**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Rank Xerox (UK) Business Services
2.9.9/3.4

## Description

The present invention relates to pyrazole compounds, and their production and use.

Organophosphorus insecticides, organochlorinated insecticides, carbamate insecticides, etc have made a great contribution in prevention and extermination of pests. Some of these insecticides, however, produce a high toxicity. Further, their residual effect causes sometimes unfavourable abnormality in the ecosystem of living things. Furthermore, resistance to those insecticides is noticed in house flies, planthoppers, leafhoppers, rice borers etc.,

Also, US-A-3 190 888 discloses new compounds represented by the Formula below:

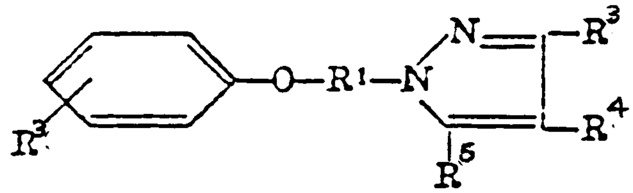

wherein $R^1$ is a divalent hydrocarbon radical having less than 8 carbon atoms in straight or branched chain configuration; $R^2$ is selected from the group consisting of hydrogen, halogen, trihaloalkyl, lower alkyl, lower alkoxy, lower alkoxyalkyl, arylalkyl, nitro, amino, dialkylamino and alkoxycarbonyl; $R^3$ and $R^5$ are selected from the group consisting of lower alkyl, halogen, lower alkoxy, lower alkoxycarbonyl, lower alkoxyalkyl, hydroxy and amino; $R^4$ is selected from the group consisting of hydrogen, halogen, lower alkyl, lower alkoxy, lower alkoxycarbonyl, lower alkoxyalkyl, hydroxy, cyano and amino. Also, Chemical Abstract, Vol.1, 109, 1988, page 257, Abstract No.50259f Discloses *inter alia* 3-methyl-1-nonyl-pyrazol and 3-methyl-1-decyl-pyrazol.

As the pesticide having a juvenile hormone-like activity, there is known "methoprene" (U.S. patent No.3 904 662). Further, Canadian Patent No.1 231 945 and EP-A1-0287959 disclose certain compounds having a juvenile hormone-like activity. However, the pesticidal activity of those compounds is not always satisfactory.

As a result of the extensive study, it has now been found that some pyrazole compounds exert a noticeable juvenile hormone-like activity and produce a remarkable pesticidal effect against pests belonging to Diptera, Hemiptera, Coleoptera, Lepidoptera, Orthoptera, Blattaria, Thysanoptera, Siphonaptera, Isoptera, etc. in agricultural fields, forest lands, granaries, stored products, sanitary facilities, etc., at low concentrations. This invention is based on the above finding.

The pyrazole compounds of the invention are representable by the formula:

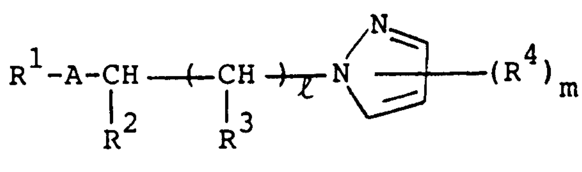

(I)

wherein

$R^1$ is a group of the formula:

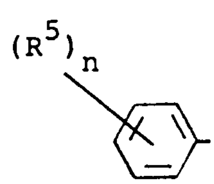

wherein $R^5$ is a hydrogen atom, a halogen atom, a hydroxy group, a cyano group, a nitro group, a $C_1$-$C_4$ alkyl group, a halo($C_1$-$C_4$)alkyl group, a $C_1$-$C_4$ alkoxy group, a halo($C_1$-$C_4$)alkoxy group, a $C_1$-$C_4$ alkylthio group, a halo($C_1$-$C_4$)alkylthio group, a $C_2$-$C_4$ alkenyl group, a $C_2$-$C_4$ alkynyl group, a $C_2$-$C_4$ alkenyloxy group, a $C_2$-$C_4$ alkynyloxy group, a $C_2$-$C_4$

alkenylthio group, a $C_2$-$C_4$ alkynylthio group, a halo($C_2$-$C_4$)alkenyl group, a halo($C_2$-$C_4$)alkynyl group, a halo($C_2$-$C_4$)alkenyloxy group, a halo($C_2$-$C_4$)alkynyloxy group or a halo($C_2$-$C_4$)alkenylthio group and n is an integer of 1 to 5;

$R^2$ and $R^3$ are, the same or different, each a hydrogen atom, a halogen atom or a $C_1$-$C_3$ alkyl group;

$R^4$ is a hydrogen atom, a halogen atom, a $C_1$-$C_4$ alkyl group or a halo($C_1$-$C_4$)alkyl group;

A is

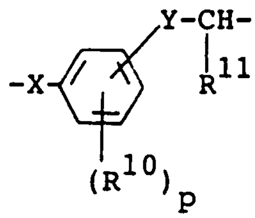

wherein $R^{10}$ and $R^{11}$ are, the same or different, each a hydrogen atom, a halogen atom or a $C_1$-$C_3$ alkyl group, X is an oxygen atom, a sulfur atom, a methylene group, a carbonyl group, a sulfoxide group, a sulfonyl group or a single bond, Y is an oxygen atom, a sulfur atom or a methylene group and p is an integer of 1 to 4;

$\ell$ is an integer of 0 to 2; and

m is an integer of 1 to 3.

The pyrazole compound (I) can be produced, for instance, by reacting a compound of the formula:

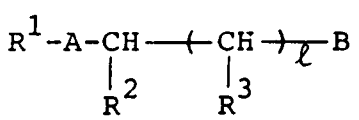 (II)

wherein $R^1$, $R^2$, $R^3$, A and $\ell$ are each as defined above and B is a halogen atom, a mesyloxy group or a tosyloxy group with a compound of the formula:

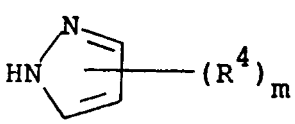 (III)

wherein $R^4$ and m are each as defined above in the presence of an acid-eliminating agent.

The above reaction may be carried out in the presence or absence of an inert solvent, of which preferred examples are dimethylformamide, dimethylsulfoxide, tetrahydrofuran, toluene, dimethoxyethane, dimethylacetamide, etc. As the acid-eliminating agent, there may be employed an alkali metal, an alkali metal hydride, an alkali metal amide, an alkali metal hydroxide, an alkali metal carbonate, an organic base (e.g. 4-dimethylaminopyridine), etc. For acceleration of the reaction, a phase transfer catalyst such as benzyltriethylammonium chloride, tetra-n-butylammonium bromide or tris(3,6-dioxaheptyl)amine may be present in the reaction. In this instance, water can be used as the reaction medium.

The reaction is normally achieved at a temperature of about -30°C to 200°C, preferably of about 0°C to 110°C, for about a period of 0.5 to 30 hours. The molar ratio of the compounds (II) and (III) is usually about 1 : 0.1 - 10 moles, preferably about 1 : 0.8 - 1.2 moles.

Upon completion of the reaction, the reaction mixture is subjected to ordinary post treatment such as extraction with an organic solvent and concentration. When desired, purification by chromatography, distillation, recrystallization or the like may be carried out.

The pyrazole compounds (I) of the invention include optical isomers and geometrical isomers with respect to $R^2$, $R^3$, and/or $R^{11}$. All of these isomers are included within the scope of the invention.

Representative examples of the pyrazole compounds (I) obtainable by the above procedure are shown in Table 1.

Table 1

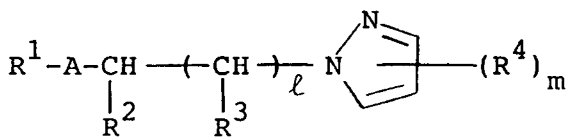

(I)

| $R^1$ | A | 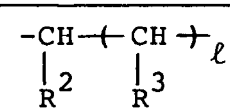 | $(R^4)_m$ |
|---|---|---|---|
| 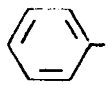 | 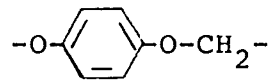 | 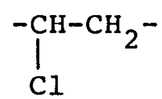 | H |
| 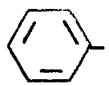 | 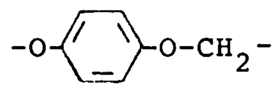 | 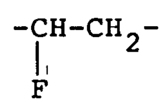 | H |
| 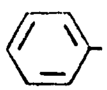 | 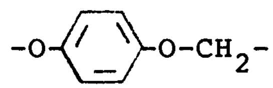 | $-CH_2-CH_2-$ | $3-C_2H_5$ |

(Continued)

| $R^1$ | A | 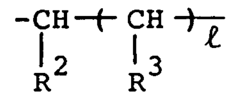 | $(R^4)_m$ |
|---|---|---|---|
| 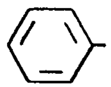 | 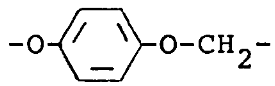 | 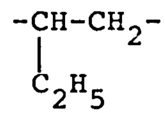 | H |
| 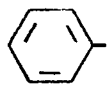 | 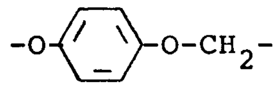 | $-CH_2-CH_2-$ | $3-CF_3$ |
| 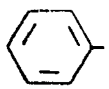 | 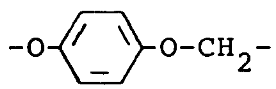 | $-CH_2-$ | $3-CH_3$ |
| 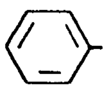 | 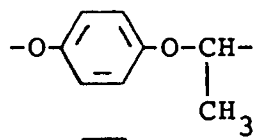 | $-CH_2-$ | H |
| 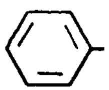 | 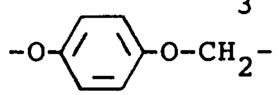 | $-CH_2-CH_2-$ | H |
| 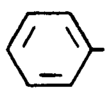 | 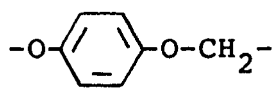 | $-CH_2-$ | H |
| 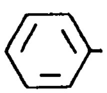 | 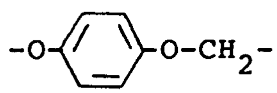 | $-CH_2-CH_2-CH_2-$ | H |

(Continued)

| $R^1$ | A | $-\underset{R^2}{CH}-(\underset{R^3}{CH})_{\ell}-$ | $(R^4)_m$ |
|---|---|---|---|
| 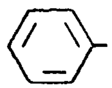 | 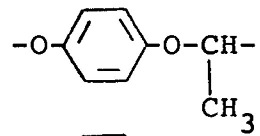 | $-CH_2-CH_2-$ | H |
| 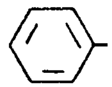 | 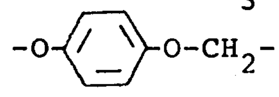 | $-\underset{CH_3}{CH}-CH_2-$ | H |
| 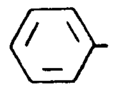 | 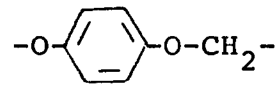 | $-CH_2-\underset{CH_3}{CH}-$ | H |
| 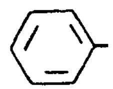 | 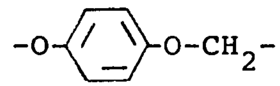 | $-CH_2-CH_2-$ | $3-CH_3$ |
| 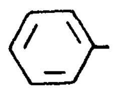 | 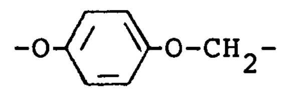 | $-CH_2-CH_2-$ | $4-CH_3$ |
| 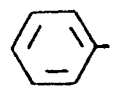 | 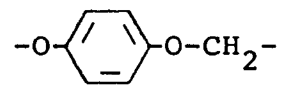 | $-CH_2-CH_2-$ | 4-Cl |
| 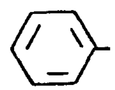 | 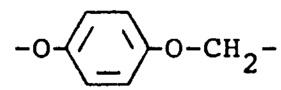 | $-CH_2-CH_2-$ | $3,5-(CH_3)_2$ |

(Continued)

| $R^1$ | A | $-\underset{R^2}{CH}-(\underset{R^3}{CH})_\ell-$ | $(R^4)_m$ |
|---|---|---|---|
| 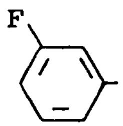 | 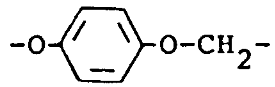 | $-CH_2-CH_2-$ | H |
| 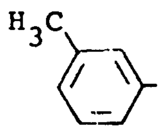 | 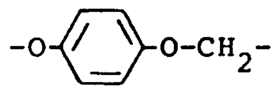 | $-CH_2-CH_2-$ | H |
| 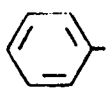 | 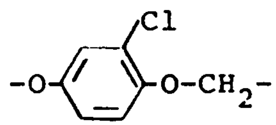 | $-CH_2-CH_2-$ | H |
| 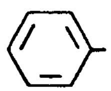 | 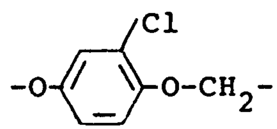 | $-CH_2-$ | H |
| 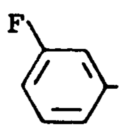 | 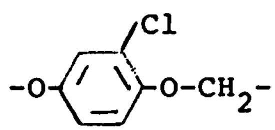 | $-CH_2-CH_2-$ | H |

(Continued)

| $R^1$ | A | $-\underset{R^2}{CH}-(\underset{R^3}{CH})_\ell-$ | $(R^4)_m$ |
|---|---|---|---|
| 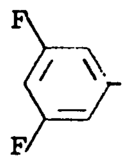 | 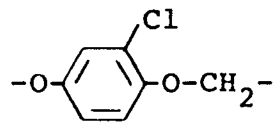 | $-CH_2-CH_2-$ | H |
| 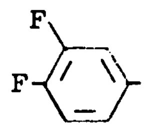 | 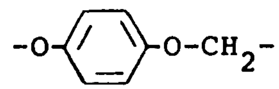 | $-CH_2-CH_2-$ | H |
| 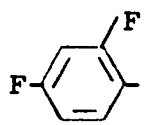 | 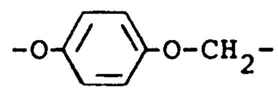 | $-CH_2-CH_2-$ | H |
| 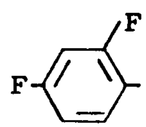 | 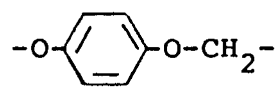 | $-CH_2-$ | H |
| 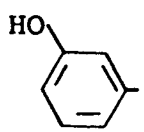 | 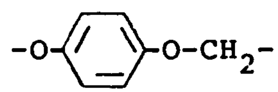 | $-CH_2-CH_2-$ | H |

(Continued)

| $R^1$ | A | $-\underset{R^2}{CH}-(\underset{R^3}{CH})_{\ell}-$ | $(R^4)_m$ |
|---|---|---|---|
| 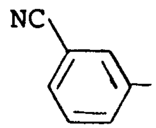 | 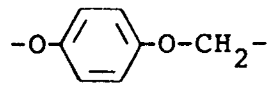 | $-CH_2-CH_2-$ | H |
| 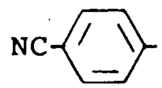 | 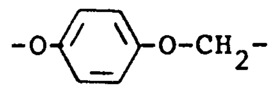 | $-CH_2-CH_2-$ | H |
| 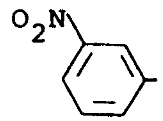 | 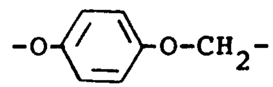 | $-CH_2-CH_2-$ | H |
| 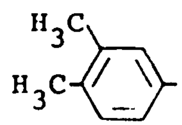 | 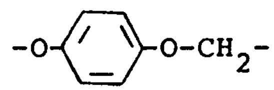 | $-CH_2-CH_2-$ | H |
| 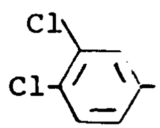 | 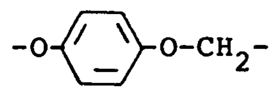 | $-CH_2-CH_2-$ | H |

(Continued)

| $R^1$ | A | 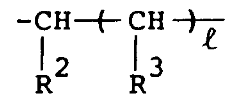 | $(R^4)_m$ |
|---|---|---|---|
| 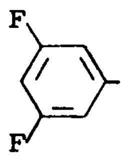 | 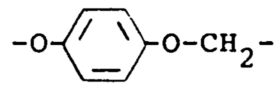 | $-CH_2-CH_2-$ | H |
| 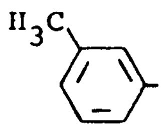 | 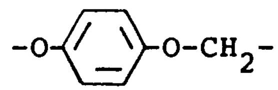 | $-CH_2-$ | H |
| 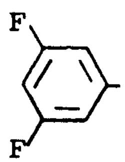 | 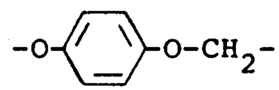 | $-CH_2-$ | H |
| 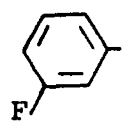 | 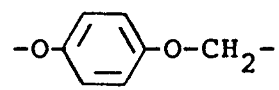 | $-CH_2-$ | H |
| 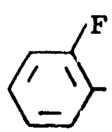 | 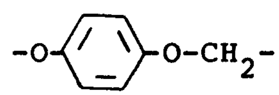 | $-CH_2-CH_2-$ | H |

(Continued)

| $R^1$ | A | $-\underset{R^2}{CH}-(\underset{R^3}{CH})_{\ell}$ | $(R^4)_m$ |
|---|---|---|---|
| 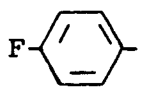 | 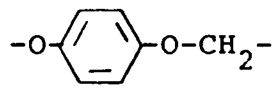 | $-CH_2-CH_2-$ | H |
| 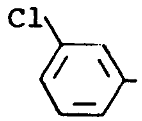 | 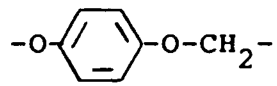 | $-CH_2-CH_2-$ | H |
| 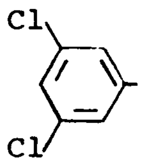 | 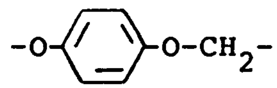 | $-CH_2-CH_2-$ | H |
| 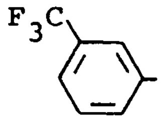 | 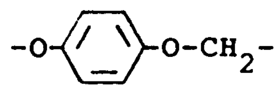 | $-CH_2-CH_2-$ | H |
| 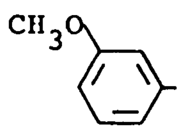 | 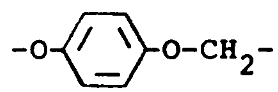 | $-CH_2-CH_2-$ | H |

(Continued)

| $R^1$ | A | $-\underset{R^2}{CH}-(\underset{R^3}{CH})_{\ell}-$ | $(R^4)_m$ |
|---|---|---|---|
| 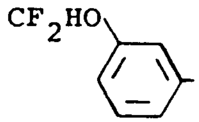 | 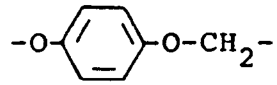 | $-CH_2-CH_2-$ | H |
| 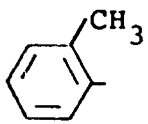 | 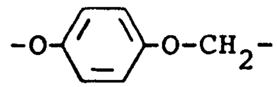 | $-CH_2-CH_2-$ | H |
| 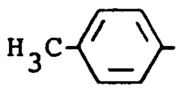 | 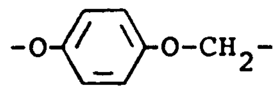 | $-CH_2-CH_2-$ | H |
| 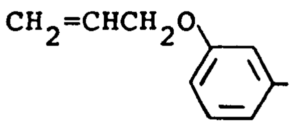 | 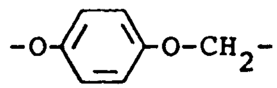 | $-CH_2-CH_2-$ | H |
| 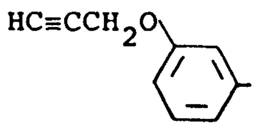 | 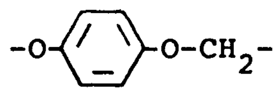 | $-CH_2-CH_2-$ | H |
| 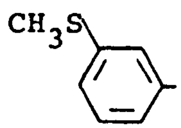 | 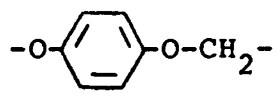 | $-CH_2-CH_2-$ | H |

(Continued)

| $R^1$ | A | $-\underset{R^2}{CH}-(\underset{R^3}{CH})_\ell-$ | $(R^4)_m$ |
|---|---|---|---|
| 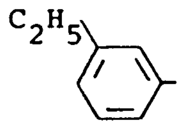 | 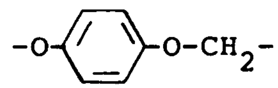 | $-CH_2-CH_2-$ | H |
| 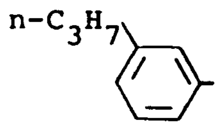 | 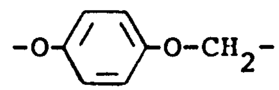 | $-CH_2-CH_2-$ | H |
| 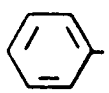 | 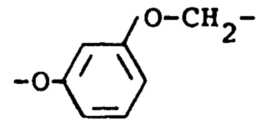 | $-CH_2-CH_2-$ | H |
| 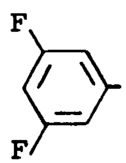 | 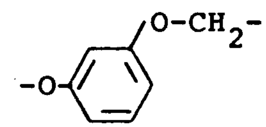 | $-CH_2-CH_2-$ | H |
| 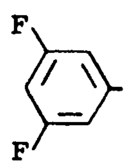 | 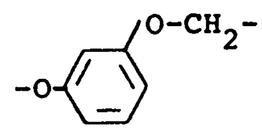 | $-CH_2-$ | H |

(Continued)

| $R^1$ | A | 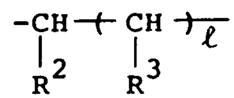 | $(R^4)_m$ |
|---|---|---|---|
| 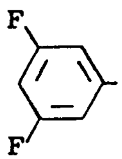 | 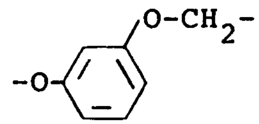 | 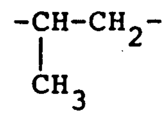 | H |
| 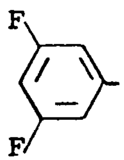 | 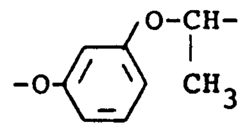 | $-CH_2-$ | H |
| 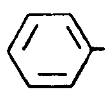 | 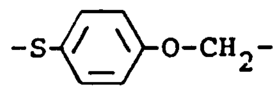 | $-CH_2-CH_2-$ | H |
| 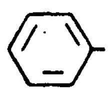 | 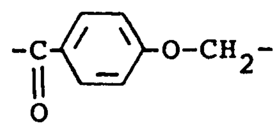 | $-CH_2-CH_2-$ | H |
| 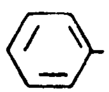 | 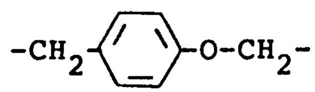 | $-CH_2-CH_2-$ | H |
| 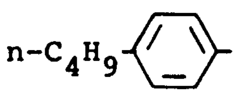 | 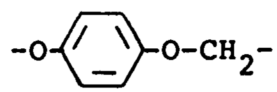 | $-CH_2-$ | H |

(Continued)

| $R^1$ | A | 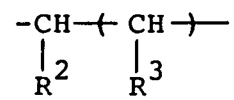 | $(R^4)_m$ |
|---|---|---|---|
| 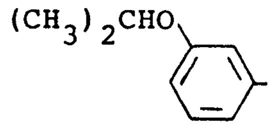 | 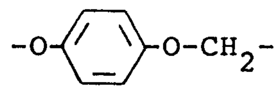 | $-CH_2-CH_2-$ | H |
| 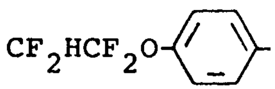 | 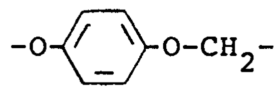 | $-CH_2-CH_2-$ | H |
| 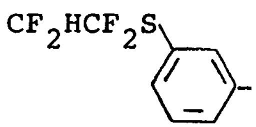 | 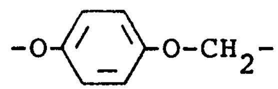 | $-CH_2-CH_2-$ | H |
| 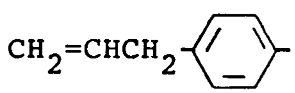 | 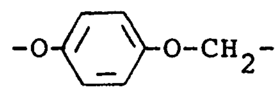 | $-CH_2-$ | H |
| 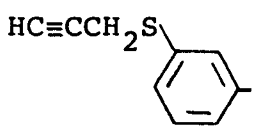 | 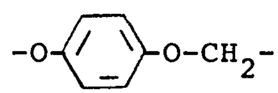 | $-CH_2-$ | H |

(Continued)

| $R^1$ | A | 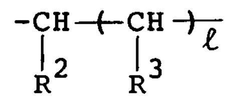 | $(R^4)_m$ |
|---|---|---|---|
| 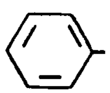 | 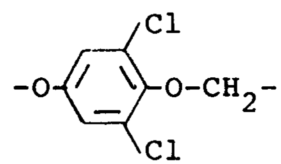 | $-CH_2-CH_2-$ | H |

(Continued)

| $R^1$ | A | 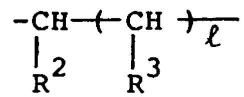 | $(R^4)_m$ |
|---|---|---|---|
| 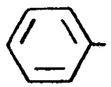 | 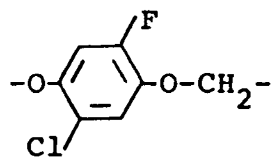 | $-CH_2-CH_2-$ | H |
| 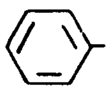 | 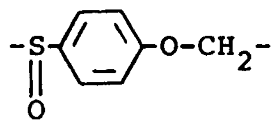 | $-CH_2-CH_2-$ | H |
| 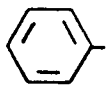 | 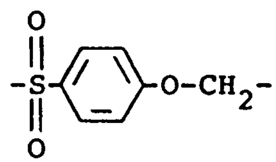 | $-CH_2-CH_2-$ | H |
| 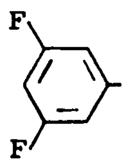 | 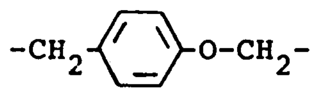 | $-CH_2-CH_2-$ | H |

(Continued)

| $R^1$ | A | $-\underset{R^2}{CH}-(\underset{R^3}{CH})_{\ell}-$ | $(R^4)_m$ |
|---|---|---|---|
| 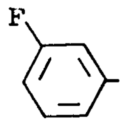 | 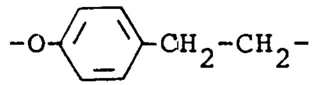 | $-CH_2-CH_2-$ | H |
| 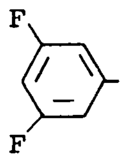 | 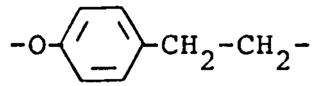 | $-CH_2-CH_2-$ | H |
| 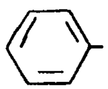 | 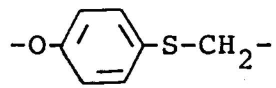 | $-CH_2-CH_2-$ | H |

(Continued)

| $R^1$ | A | $-\underset{R^2}{CH}-(\underset{R^3}{CH})_{\ell}-$ | $(R^4)_m$ |
| --- | --- | --- | --- |
| 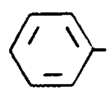 | $-O-(CH_2)_5-$ | $-CH_2-CH_2-$ | H |
| 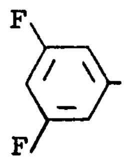 | $-O-(CH_2)_5-$ | $-CH_2-CH_2-$ | H |

(Continued)

| $R^1$ | A | 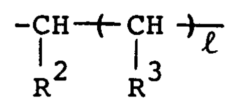 | $(R^4)_m$ |
|---|---|---|---|
| 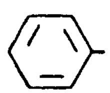 | 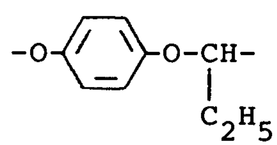 | $-CH_2-CH_2-$ | H |

(Continued)

| $R^1$ | A | 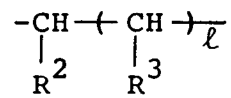 | $(R^4)_m$ |
|---|---|---|---|
| 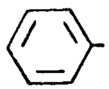 | 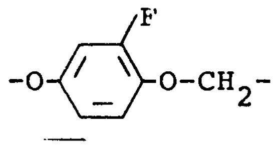 | $-CH_2-CH_2-$ | H |
| 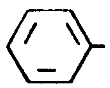 | 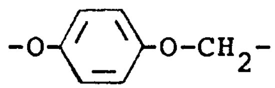 | 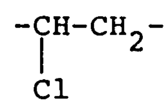 | H |
| 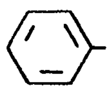 | 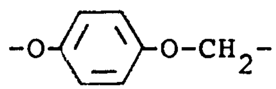 | 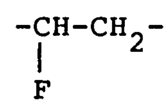 | H |
| 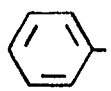 | 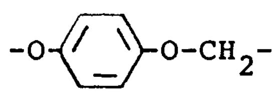 | $-CH_2-CH_2-$ | $3-C_2H_5$ |

(Continued)

| $R^1$ | A | 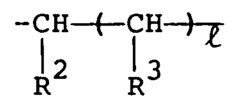 | $(R^4)_m$ |
|---|---|---|---|
| 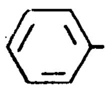 | 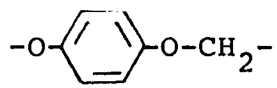 | 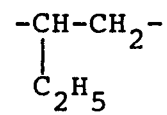 | H |
| 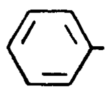 | 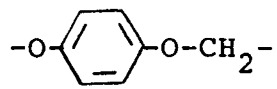 | $-CH_2-CH_2-$ | $3-CF_3$ |
| 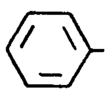 | 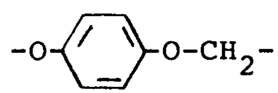 | $-CH_2-$ | $3-CH_3$ |
| 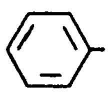 | 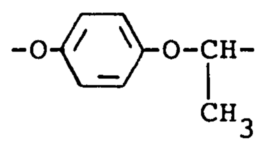 | $-CH_2-$ | H |

(Continued)

| $R^1$ | A | 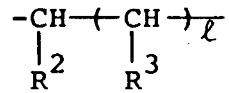 | $(R^4)_m$ |
|---|---|---|---|
| 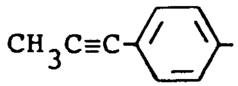 | 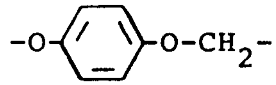 | $-CH_2-CH_2-$ | H |
| 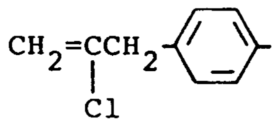 | 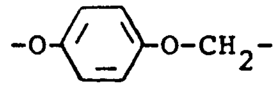 | $-CH_2-CH_2-$ | H |
| 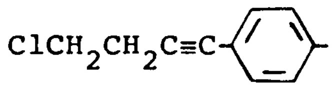 | 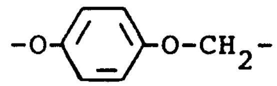 | $-CH_2-CH_2-$ | H |
| 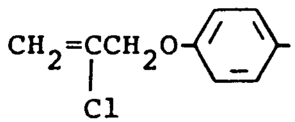 | 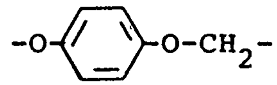 | $-CH_2-CH_2-$ | H |
| 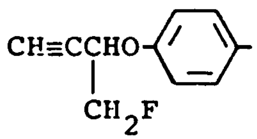 | 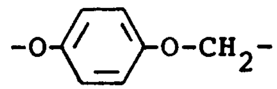 | $-CH_2-CH_2-$ | H |
| 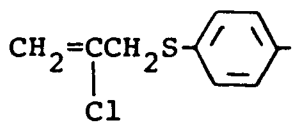 | 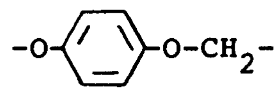 | $-CH_2-CH_2-$ | H |
| 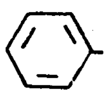 | $-O-(CH_2)_3-CH=CH-$ | $-CH_2-CH_2-$ | H |

(Continued)

| $R^1$ | A | $-CH(R^2)-(CH(R^3))_\ell-$ | $(R^4)_m$ |
|---|---|---|---|
| 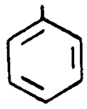 | $-S-(CH_2)_5-$ | $-CH_2-CH_2-$ | H |
| 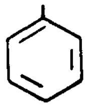 | $-O-(CH_2)_3-CH=C(CH_3)-$ | $-CH_2-CH_2-$ | H |
| 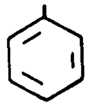 | $-O-(CH_2)_3-CH=C(F)-$ | $-CH_2-CH_2-$ | H |
| 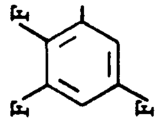 | -O-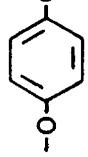-O-$CH_2$- | $-CH_2-CH_2-$ | H |
| 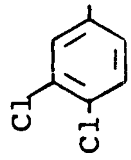 | -O-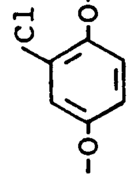-O-$CH_2$- | $-CH_2-CH_2-$ | H |
| 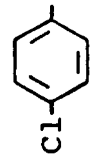 | -O-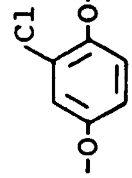-O-$CH_2$- | $-CH_2-CH_2-$ | H |

Practical and presently preferred embodiments for preparation of the pyrazole compounds (I) are illustratively shown in the following examples.

Example 1

To a mixture of anhydrous N,N-dimethylformamide (5 ml) and sodium hydride (60 % oil suspension; 39 mg), pyrazole (66 mg) was added, and the resultant mixture was stirred for 30 minutes. A solution of 3-(4-phenoxyphenoxy)propyl bromide (300 mg) in anhydrous N,N-dimethylformamide (5 ml) was dropwise added thereto, followed by stirring at room temperature for 5 hours. The reaction mixture was diluted with ethyl acetate (50 ml), washed with a saturated aqueous ammonium chloride solution two times, dried over anhydrous magnesium sulfate and concentrated under reduced pressure. The oily substance thus obtained was subjected to column chromatography to give 246 mg of 1-[3-(4-phenoxyphenoxy)propyl]pyrazole as a colorless oil. $n_D^{23.5}$ 1.5777.

Example 2

To a mixture of anhydrous dimethylsulfoxide (5 ml) and sodium hydride (60 % oil suspension; 62 mg), pyrazole (106 mg) was added, and the resultant mixture was stirred for 30 minutes. A solution of 3-[4-(3-tolyloxy)phenoxy]propyl bromide (500 mg) in anhydrous dimethylsulfoxide (5 ml) was dropwise added thereto, followed by stirring at room temperature for 3 hours. The reaction mixture was treated in the same manner as in Example 1 to give 347 mg of 1-{3-[4-(3-tolyloxy)phenoxy]propyl}pyrazole as a colorless oil. $n_D^{23.7}$ 1.5738.

In the same manner as above, there were prepared the pyrazole compounds (I), of which typical examples are shown in Table 2.

Table 2

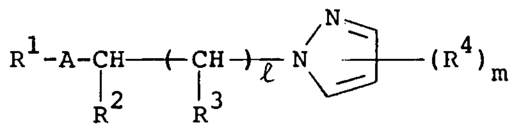

| Compound No. | $R^1$ | A | 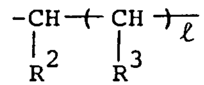 | $(R^4)_m$ | Physical constant |
|---|---|---|---|---|---|
| 1 | 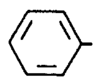 | 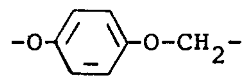 | $-CH_2-CH_2-$ | H | $n_D^{23.5}$ 1.5777 |
| 2 | 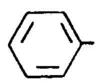 | 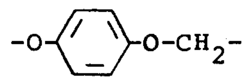 | $-CH_2-$ | H | m.p., 59.7°C |
| 3 | 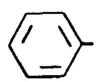 | 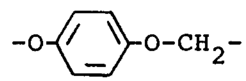 | $-CH_2-CH_2-CH_2-$ | H | $n_D^{24.7}$ 1.5731 |
| 5 | 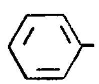 | 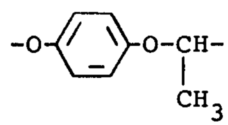 | $-CH_2-CH_2-$ | H | $n_D^{24.5}$ 1.5799 |

(Continued)

| Compound No. | $R^1$ | A | $-CH(R^2)-(CH(R^3))_\ell-$ | $(R^4)_m$ | Physical constant |
|---|---|---|---|---|---|
| 6 | 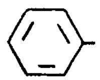 | 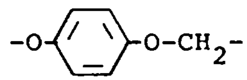 | $-CH(CH_3)-CH_2-$ | H | $n_D^{24.5}$ 1.5795 |
| 7 | 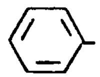 | 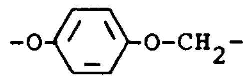 | $-CH_2-CH(CH_3)-$ | H | $n_D^{24.3}$ 1.5801 |
| 8 | 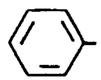 | 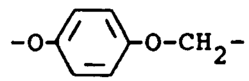 | $-CH_2-CH_2-$ | $3-CH_3$ | $n_D^{24.5}$ 1.5705 |
| 9 | 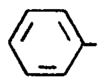 | 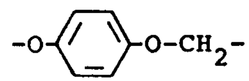 | $-CH_2-CH_2-$ | $4-CH_3$ | $n_D^{23.5}$ 1.5691 |
| 10 | 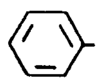 | 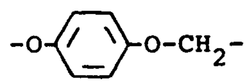 | $-CH_2-CH_2-$ | 4-Cl | $n_D^{24.3}$ 1.5825 |
| 11 | 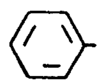 | 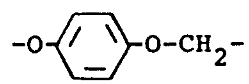 | $-CH_2-CH_2-$ | $3,5-(CH_3)_2$ | $n_D^{25.3}$ 1.5648 |
| 12 | 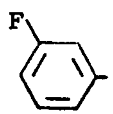 | 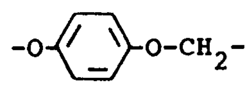 | $-CH_2-CH_2-$ | H | $n_D^{24.2}$ 1.5761 |

(Continued)

| Compound No. | $R^1$ | A | $-\underset{R^2}{CH}-(\underset{R^3}{CH})_\ell-$ | $(R^4)_m$ | Physical constant |
|---|---|---|---|---|---|
| 13 | 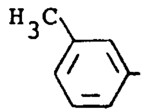 | 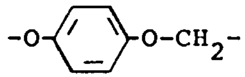 | $-CH_2-CH_2-$ | H | $n_D^{23.7}$ 1.5738 |
| 14 | 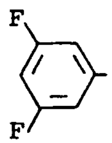 | 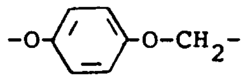 | $-CH_2-CH_2-$ | H | $n_D^{23.5}$ 1.5746 |
| 15 | 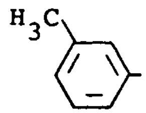 | 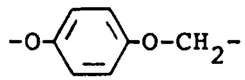 | $-CH_2-$ | H | $n_D^{23.8}$ 1.5793 |
| 16 | 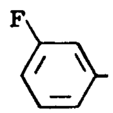 | 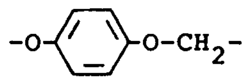 | $-CH_2-$ | H | $n_D^{23.7}$ 1.5802 |
| 17 | 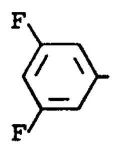 | 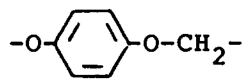 | $-CH_2-$ | H | $n_D^{23.3}$ 1.5798 |

(Continued)

| Compound No. | $R^1$ | A | $-CH(R^2)-(CH(R^3))_\ell-$ | $(R^4)_m$ | Physical constant |
|---|---|---|---|---|---|
| 18 | 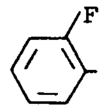 | 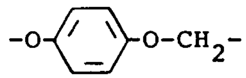 | $-CH_2-CH_2-$ | H | $n_D^{23.5}$ 1.5775 |
| 19 | 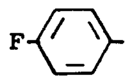 | 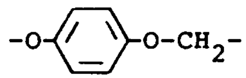 | $-CH_2-CH_2-$ | H | $n_D^{24.1}$ 1.5765 |
| 20 | 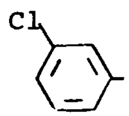 | 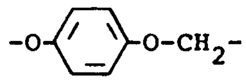 | $-CH_2-CH_2-$ | H | $n_D^{24.5}$ 1.5786 |
| 21 | 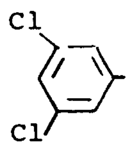 | 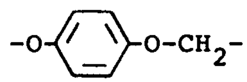 | $-CH_2-CH_2-$ | H | $n_D^{24.3}$ 1.5794 |
| 22 | 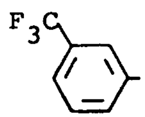 | 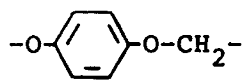 | $-CH_2-CH_2-$ | H | $n_D^{23.5}$ 1.5631 |
| 23 | 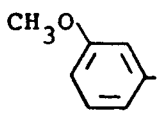 | 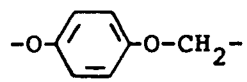 | $-CH_2-CH_2-$ | H | $n_D^{24.3}$ 1.5772 |

(Continued)

| Compound No. | $R^1$ | A | $-CH(R^2)-(CH(R^3))_\ell-$ | $(R^4)_m$ | Physical constant |
|---|---|---|---|---|---|
| 24 | 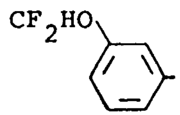 | 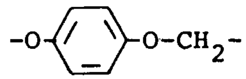 | $-CH_2-CH_2-$ | H | $n_D^{24.0}$ 1.5649 |
| 25 | 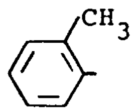 | 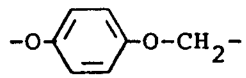 | $-CH_2-CH_2-$ | H | $n_D^{24.0}$ 1.5769 |
| 26 | 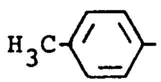 | 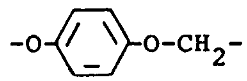 | $-CH_2-CH_2-$ | H | $n_D^{23.4}$ 1.5761 |
| 27 | 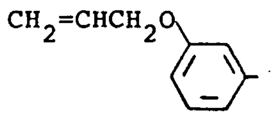 | 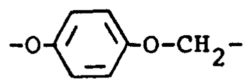 | $-CH_2-CH_2-$ | H | $n_D^{24.0}$ 1.5815 |
| 28 | 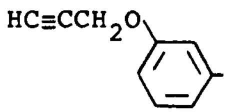 | 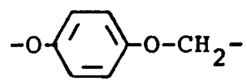 | $-CH_2-CH_2-$ | H | $n_D^{23.4}$ 1.5796 |
| 29 | 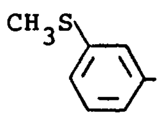 | 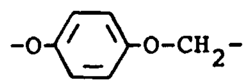 | $-CH_2-CH_2-$ | H | $n_D^{23.5}$ 1.5850 |

(Continued)

| Compound No. | $R^1$ | A | $-\underset{R^2}{CH}-(\underset{R^3}{CH})_\ell$ | $(R^4)_m$ | Physical constant |
|---|---|---|---|---|---|
| 30 | 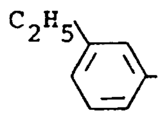 | 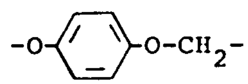 | $-CH_2-CH_2-$ | H | $n_D^{24.2}$ 1.5756 |
| 31 | 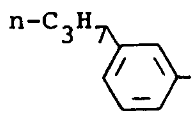 | 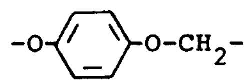 | $-CH_2-CH_2-$ | H | $n_D^{24.1}$ 1.5765 |
| 32 | 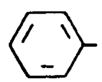 | 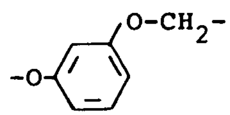 | $-CH_2-CH_2-$ | H | $n_D^{24.5}$ 1.5781 |
| 33 | 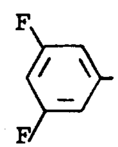 | 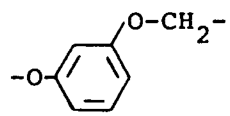 | $-CH_2-CH_2-$ | H | $n_D^{23.5}$ 1.5786 |
| 34 | 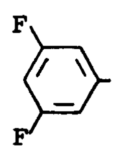 | 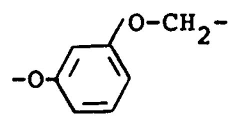 | $-CH_2-$ | H | $n_D^{24.1}$ 1.5803 |

(Continued)

| Compound No. | $R^1$ | A | $-CH(R^2)-(CH(R^3))_\ell-$ | $(R^4)_m$ | Physical constant |
|---|---|---|---|---|---|
| 35 | 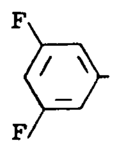 | 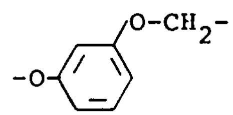 | $-CH-CH_2-$<br>$CH_3$ | H | $n_D^{24.0}$ 1.5795 |
| 36 | 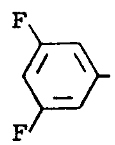 | 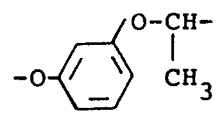 | $-CH_2-$ | H | $n_D^{24.5}$ 1.5812 |
| 37 | 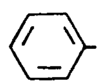 | 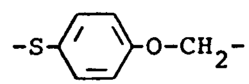 | $-CH_2-CH_2-$ | H | $n_D^{25.0}$ 1.5860 |
| 38 | 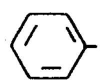 | 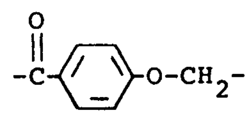 | $-CH_2-CH_2-$ | H | $n_D^{24.2}$ 1.5798 |
| 39 | 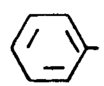 | 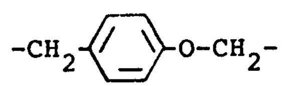 | $-CH_2-CH_2-$ | H | $n_D^{24.7}$ 1.5781 |

(Continued)

| Compound No. | $R^1$ | A | $-CH(R^2)-(CH(R^3))_\ell-$ | $(R^4)_m$ | Physical constant |
|---|---|---|---|---|---|
| 40 | 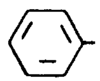 | 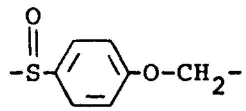 | $-CH_2-CH_2-$ | H | $n_D^{25.1}$ 1.5881 |
| 41 | 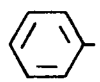 | 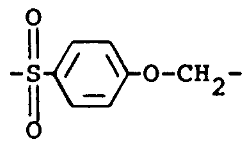 | $-CH_2-CH_2-$ | H | $n_D^{24.7}$ 1.5834 |
| 42 | 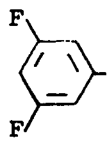 | 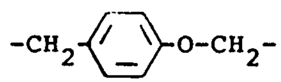 | $-CH_2-CH_2-$ | H | $n_D^{24.8}$ 1.5773 |
| 43 | 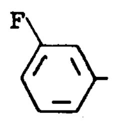 | 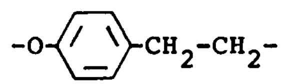 | $-CH_2-CH_2-$ | H | $n_D^{24.5}$ 1.5726 |
| 44 | 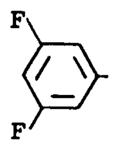 | 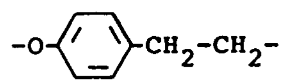 | $-CH_2-CH_2-$ | H | $n_D^{24.6}$ 1.5745 |

(Continued)

| Compound No. | $R^1$ | A | $-\underset{R^2}{CH}-(-\underset{R^3}{CH}-)_{\ell}$ | $(R^4)_m$ | Physical constant |
|---|---|---|---|---|---|
| 45 | 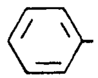 | -O-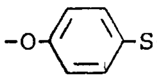-S-$CH_2$- | $-CH_2-CH_2-$ | H | $n_D^{24.2}$ 1.5817 |
| 50 | 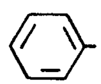 | $-O-(CH_2)_5-$ | $-CH_2-CH_2-$ | H | $n_D^{24.5}$ 1.5325 |

(Continued)

| Compound No. | $R^1$ | A | $-\underset{R^2}{CH}-(\underset{R^3}{CH})_{\ell}$ | $(R^4)_m$ | Physical constant |
|---|---|---|---|---|---|
| 51 | 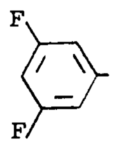 | $-O-(CH_2)_5-$ | $-CH_2-CH_2-$ | H | $n_D^{24.5}$ 1.5331 |

(Continued)

| Compound No. | $R^1$ | A | $-CH(R^2)-(CH(R^3))_\ell-$ | $(R^4)_m$ | Physical constant |
|---|---|---|---|---|---|
| 63 | 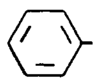 | 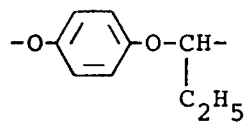 | $-CH_2-CH_2-$ | H | $n_D^{24.7}$ 1.5799 |
| 65 | 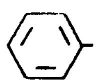 | 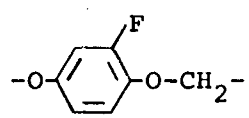 | $-CH_2-CH_2-$ | H | $n_D^{24.2}$ 1.5775 |
| 67 | 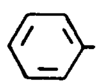 | 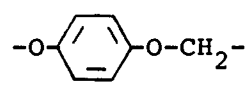 | $-CH(Cl)-CH_2-$ | H | $n_D^{24.5}$ 1.5781 |

(Continued)

| Compound No. | $R^1$ | A | $-\underset{R^2}{CH}-(\underset{R^3}{CH})_{\ell}-$ | $(R^4)_m$ | Physical constant |
|---|---|---|---|---|---|
| 68 | 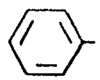 | 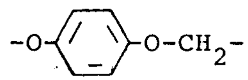 | $-\underset{F}{CH}-CH_2-$ | H | $n_D^{24.5}$ 1.5779 |
| 70 | 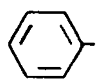 | 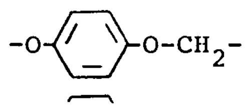 | $-CH_2-CH_2-$ | $3-C_2H_5$ | $n_D^{25.0}$ 1.5698 |
| 72 | 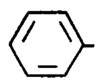 | 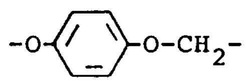 | $-\underset{C_2H_5}{CH}-CH_2-$ | H | $n_D^{23.5}$ 1.5797 |
| 73 | 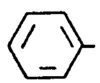 | 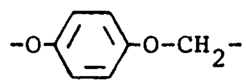 | $-CH_2-CH_2-$ | $3-CF_3$ | $n_D^{25.1}$ 1.5717 |

(Continued)

| Compound No. | $R^1$ | A | $-\underset{R^2}{CH}-(\underset{R^3}{CH})_{\ell}-$ | $(R^4)_m$ | Physical constant |
|---|---|---|---|---|---|
| 75 | 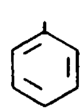 | 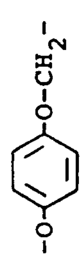 | $-CH_2-$ | $3-CH_3$ | $n_D^{24.3}$ 1.5790 |
| 76 | 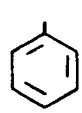 | 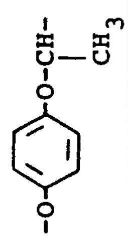 | $-CH_2-$ | H | $n_D^{24.0}$ 1.5781 |

On the application of the pyrazole compound (I) as a pesticide, it may be used as such or in an appropriate preparation form such as an oil spray, an emulsifiable concentrate, a wettable powder, granules, a dust, an aerosol, a fogging

agent, a toxic bait, etc. In those preparations, the pyrazole compound (I) is usually contained in about 0.1 to about 99.9 % by weight, preferably in about 2 to about 80 % by weight.

Said preparation can be formulated in a per se conventional manner by mixing at least one of the pyrazole compounds (I) with an appropriate solid, liquid or gaseous carrier(s) or diluent(s) or a bait. An appropriate adjuvant(s) such as a surfactant, an adherent, a dispersant or a stabilizer may be also mixed therein for improving the dispersibility and other properties of the preparation.

Examples of the solid carriers or diluents are fine powders or granules of clays (e.g. kaolin clay, diatomaceous earth, synthetic hydrated silica, bentonite, fubasami clay, terra alba), talcs, ceramics, other inorganic minerals (e.g. sericite, quartz, sulfur, active carbon, calcium carbonate, hydrated silica), chemical fertilizers (e.g. ammonium sulfate, ammonium phosphate, ammonium nitrate, urea, ammonium chloride), etc. Examples of the liquid carriers or diluents are water, alcohols (e.g. methanol, ethanol), ketones (e.g. acetone, methylethylketone), aromatic hydrocarbons (e.g. benzene, toluene, xylene, ethylbenzene, methylnaphthalene), aliphatic hydrocarbons (e.g. hexane, cyclohexane, kerosene, light oil), esters (e.g. ethyl acetate, butyl acetate), nitriles (e.g. acetonitrile, isobutyronitrile), ethers (e.g. diisopropyl ether, dioxane), acid amides (e.g. N,N-dimethylformamide, N,N-dimethylacetamide), halogenated hydrocarbons (e.g. dichloromethane, trichloroethane, carbon tetrachloride), dimethylsulfoxide, botanical oils (e.g. soybean oil, cotton-seed oil), etc. Examples of the gaseous carriers or diluents are Freon gas, butane gas, LPG (liquefied petroleum gas), dimethyl ether, carbon dioxide, etc.

The surfactants usable for emulsification, dispersion or spreading may be any of ionic and non-ionic types. Their examples are alkylsulfates, alkylarylsulfonates, dialkylsulfosuccinates, polyoxyethylenealkylarylphosphates, condensates of naphthalenesulfonic acid and formalin, polyoxyethylene alkyl ethers, polyoxyethylene polyoxypropylene block copolymers, sorbitan fatty acid esters, polyoxyethylene sorbitan fatty acid esters, etc. Examples of the adherents or dispersants may include casein, gelatin, polyvalent alcohols (e.g. starch powder, gum arabic, cellulose derivatives, alginic acid), lignin derivatives, bentonite, saccharides, synthetic water-soluble high molecular compounds (e.g. polyvinyl alcohol, polyvinylpyrrolidone, polyacrylic acid), etc. As the stabilizers, there may be used alkyl phosphates (e.g. PAP (isopropyl acid phosphate), BHT (2,6-di-tert-butyl-4-methyl-phenol), BHA (a mixture of 2-tert-butyl-4-methoxyphenol and 3-tert-butyl-4-methoxyphenol), botanical oils, mineral oils, surfactants, aliphatic acids or esters, etc.

The base for toxic baits may comprise food (e.g. grain powders, essential oils, sugar, crystalline cellulose), an antioxidant (e.g. dibutylhydroxytoluene, butylhydroxyanisole, nordihydroguaiaretic acid), a preservative (e.g. dehydroacetic acid), a mis-feed inhibitor (e.g. red pepper powders), a flavoring agent (e.g. cheese flavor, onion flavor), etc.

The composition thus formulated may be applied as such or in a form of dilution with water. In addition, said composition may contain other insecticides, nematocides, acaricides, fungicides, herbicides, plant growth regulators, synergistic agents, fertilizers, soil improvers, etc. Particularly when employed in conjunction with conventional insecticides, a broad spectrum of activity or a more immediate effect on very heterogeneous populations is provided. Examples of the insecticides include organic phosphorus compounds (e.g. fenitrothion (O,O-dimethyl-O-(3-methyl-4-nitrophenyl)phosphorothioate), malathion (S-[1,2-bis(ethoxycarbonyl)ethyl] O,O-dimethylphosphorothioate), dimethoate (O,O-dimethyl-S-(N-methylcarbamoylmethyl)phosphorodithioate), salithion (2-methoxy-4H-1,3,2-benzodioxaphosphorin-2-sulfide), diazinon (O,O-diethyl-O-(2-isopropyl-6-methyl-4-pyrimidinyl)phosphorothioate), dipterex (2,2,2-trichloro-1-hydroxyethyl-O,O-dimethylphosphonate), dichlorvos (O-(2,2-dichlorovinyl)-O,O-dimethylphosphate), etc.), carbamate compounds (e.g. MPMC (3,4-dimethylphenyl N-methylcarbamate), MTMC (m-tolyl N-methylcarbamate), BPMC (2-sec-butylphenyl N-methylcarbamate), carbaryl (1-naphthyl N-methylcarbamate), etc.) and pyrethroid compounds (e.g. permethrin (3-phenoxybenzyl-*d*,*ℓ*-cis,trans-3-(2,2-dichlorovinyl)-2,2-dimethylcyclopropanecarboxylate), fenvalerate (α-cyano-m-phenoxybenzyl α-isopropyl-p-chlorophenylacetate), etc.).

The composition may be applied to pests by a conventional manner, of which typical examples are spreading, fuming, soil treatment, incorporation into food for domestic animals or poultry, etc. It is further noticeable that addition of the composition to sericiculture food may lead to an increase of cocoons in number or thickening the cocoon layer.

The dosage of the pyrazole compound (I) as the active ingredient in an agricultural pesticidal composition is generally from about 5 to about 500 grams per 100 ares. When the composition is applied as an emulsifiable concentrate or a wettable powder, the concentration of the active ingredient is normally from about 1 to about 500 ppm. In case of such formulation as granules, fine granules and dusts, the composition may be applied as such without diluting with water. As a sanitary pesticidal composition, the composition in the form of an emulsifiable concentrate, an emulsifiable concentrate or a wettable powder may be diluted with water in a concentration of the active ingredient being generally from about 1 to about 500 ppm and applied. In case of the formulation such as an oil spray, an aerosol, a fumigant, a bait or the like, it may be applied as such.

Said amounts and concentrations are not decisive and may vary depending on the kind of preparation, season for application, locus to be applied, mode of application, species of pests, degree of damages, etc.

Some practical embodiments of the composition according to the invention are illustratively shown in the following Formulation Examples wherein % and part(s) are by weight and the compound numbers correspond to those in Table 2.

Formulation Example 1

One of Compound Nos. 1 to 76 (20 parts), an emulsifier (a mixture of polyoxyethylene styrylphenyl ether, polymer of polyoxyethylene styrylphenyl ether and alkylarylsulfonate) (20 parts) and xylene (60 parts) are mixed well to make a 20 % emulsifiable concentrate.

Formulation Example 2

One of Compound Nos. 1 to 76 (20 parts), an emulsifier (sodium laurylsulfate) (5 parts) and diatomaceous earth (#300 mesh; 75 parts) are mixed well in a pulverizer to make a 20 % wettable powder.

Formulation Example 3

One of Compound Nos. 1 and 2 (3 parts), acetone (20 parts) and talc (#300 mesh; 97 parts) are mixed well in a pulverizer, followed by removal of acetone by evaporation to make a 3 % dust.

Formulation Example 4

One of Compound Nos. 1 to 76 (5 parts), a dispersing agent (calcium ligninsulfonate) (2 parts) and clay (93 parts) are mixed well, followed by addition of a small amount of water. The resultant mixture is kneaded and granulated by the aid of a granulator and dried to make 5 % granules.

Formulation Example 5

Compound No. 1 (2 parts), a dispersing agent (calcium ligninsulfonate) (2 parts) and clay (96 parts) are mixed well, followed by addition of a small amount of water. The resultant mixture is kneaded and granulated by the aid of a fine granulator and dried to make 2 % fine granules.

Formulation Example 6

Compound No. 1 (0.2 parts), xylene (2 parts), dimethylformamide (2 parts) and lamp oil (95.8 parts) are mixed well to make an oil spray.

Formulation Example 7

Compound No. 1 (0.05 part), tetramethrin (N-(3,4,5,6-tetrahydrophthalimido)methylchrysanthemate) (0.2 part), resmethrin (5-benzyl-3-furylmethyl) (±)-cis,trans-chrysanthemate) (0.05 part), xylene (7 parts) and deodorized lamp oil (42.7 parts) are mixed well and charged into an aerosol container. Upon attachment of a valve portion, a pressurizing agent (LPG) (50 parts) is charged through the valve to make an aerosol.

Formulation Example 8

Compound No. 1 (1 part) and sesame oil (3 parts) are mixed, and butyl hydroxyanisole (0.03 part), dehydroacetic acid (0.1 part), black sugar (10 parts), crystalline cellulose (30 parts) and potato starch (55.87 parts) are added thereto. The resultant mixture is uniformly mixed and pressurized with a load of 15 $kg/cm^2$ to make a toxic bait in tablets, each tablet having a weight of approx. 4 g and a diameter of 30 mm.

The following Test Examples present some typical test data indicating the excellent pesticidal activity of the pyrazole compounds (I). The compounds used for comparison are shown in Table 3 below:

Table 3

| Compound No. | Structure | Remarks |
|---|---|---|
| (A) | 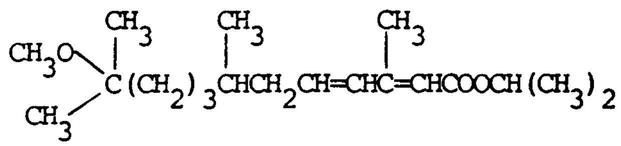 | Known as "methoprene"; U.S. patent 3,904,662 |
| (B) | 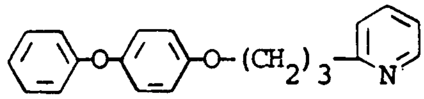 | Canadian patent 1,231,945; Compound No. 118 |
| (C) | 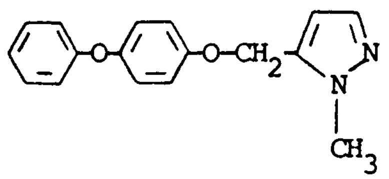 | EP-A1-287959; Compound No. 14-1 |

Test Example 1

An emulsifiable concentrate prepared according to Formulation Example 1 was diluted with water to make a 400 fold dilution. The dilution (0.7 ml) was added to 100 ml of distilled water. Last instar larvae of common mosquito (Culex pipiens pallens) were released therein and reared for 7 days until their emergence. The rate of emergence was observed

with two replications. The results are shown in Table 4.

Table 4

| Test compound No. | Concentration (ppm) | Rate of emergence (%) |
|---|---|---|
| 1 | 3.5 | 0 |
| 2 | 3.5 | 0 |
| 3 | 3.5 | 0 |
| 5 | 3.5 | 0 |
| 6 | 3.5 | 0 |
| 7 | 3.5 | 0 |
| 8 | 3.5 | 0 |
| 9 | 3.5 | 0 |
| 10 | 3.5 | 0 |
| 11 | 3.5 | 0 |
| 12 | 3.5 | 0 |
| 13 | 3.5 | 0 |
| 14 | 3.5 | 0 |
| 15 | 3.5 | 0 |
| 16 | 3.5 | 0 |
| 17 | 3.5 | 0 |
| 18 | 3.5 | 0 |
| 19 | 3.5 | 0 |
| 20 | 3.5 | 0 |
| 21 | 3.5 | 0 |
| 22 | 3.5 | 0 |
| 23 | 3.5 | 0 |
| 24 | 3.5 | 0 |
| 25 | 3.5 | 0 |
| 26 | 3.5 | 0 |
| 27 | 3.5 | 0 |
| 28 | 3.5 | 0 |
| 29 | 3.5 | 0 |
| 30 | 3.5 | 0 |
| 31 | 3.5 | 0 |
| 32 | 3.5 | 0 |
| 33 | 3.5 | 0 |
| 34 | 3.5 | 0 |
| 35 | 3.5 | 0 |
| 36 | 3.5 | 0 |
| 37 | 3.5 | 0 |
| 38 | 3.5 | 0 |
| 39 | 3.5 | 0 |
| 40 | 3.5 | 0 |
| 41 | 3.5 | 0 |
| 42 | 3.5 | 0 |
| 43 | 3.5 | 0 |
| 44 | 3.5 | 0 |
| 45 | 3.5 | 0 |

(Continued)

| Test compound No. | Concentration (ppm) | Rate of emergence (%) |
|---|---|---|
| 50 | 3.5 | 0 |
| 51 | 3.5 | 0 |
| 63 | 3.5 | 0 |
| 65 | 3.5 | 0 |
| 67 | 3.5 | 0 |
| 68 | 3.5 | 0 |
| 70 | 3.5 | 0 |
| 72 | 3.5 | 0 |
| 73 | 3.5 | 0 |
| 75 | 3.5 | 0 |
| 76 | 3.5 | 0 |
| (A) | 3.5 | 0 |
| Untreated | - | 90 |

Test Example 2

Powdered animal feed (2 g) was thoroughly mixed with bran (14 g). An emulsifiable concentrate prepared according to Formulation Example 1 was diluted with water to a designed concentration, and the dilution was added to the above mixture. The resultant mixture was stirred well to make an artificial culture. Thirty larvae of housefly (Musca domestica) were reared therein until their pupation. The obtained pupae were placed into a plastic cup, and the rate of emergence was determined. According to the following equation, the emergence inhibition (%) was calculated:

$$\text{Emergence inhibition (\%)} = \left(1 - \frac{\text{Rate of emergence in treated plot}}{\text{Rate of emergence in untreated plot}}\right) \times 100$$

The results are shown in Table 5.

Table 5

| Test compound No. | Emergence inhibition (%) | |
|---|---|---|
| | 3 ppm | 1 ppm |
| 1 | 100 | 100 |
| 2 | 100 | 88 |
| 15 | 91 | 58 |
| 76 | 100 | 88 |
| (A) | 60 | 13 |
| (B) | 40 | 0 |
| (C) | 5 | 0 |

## Claims

1. A pyrazole compound of the formula:

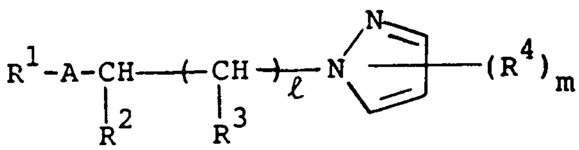

wherein

$R^1$ is a group of the formula:

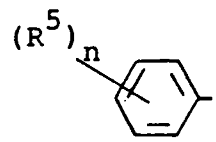

wherein $R^5$ is a hydrogen atom, a halogen atom, a hydroxy group, a cyano group, a nitro group, a $C_1$-$C_4$ alkyl group, a halo($C_1$-$C_4$)alkyl group, a $C_1$-$C_4$ alkoxy group, a halo($C_1$-$C_4$)alkoxy group, a $C_1$-$C_4$ alkylthio group, a halo($C_1$-$C_4$)alkylthio group, a $C_2$-$C_4$ alkenyl group, a $C_2$-$C_4$ alkynyl group, a $C_2$-$C_4$ alkenyloxy group, a $C_2$-$C_4$ alkynyloxy group, a $C_2$-$C_4$ alkenylthio group, a $C_2$-$C_4$ alkynylthio group, a halo($C_2$-$C_4$)alkenyl group, a halo($C_2$-$C_4$)alkynyl group, a halo($C_2$-$C_4$)alkenyloxy group, a halo($C_2$-$C_4$)alkynyloxy group or a halo($C_2$-$C_4$)alkenylthio group and n is an integer of 1 to 5;

$R^2$ and $R^3$ are, the same or different, each a hydrogen atom, a halogen atom or a $C_1$-$C_3$ alkyl group;

$R^4$ is a hydrogen atom, a halogen atom, a $C_1$-$C_4$ alkyl group or a halo($C_1$-$C_4$)alkyl group;

A is

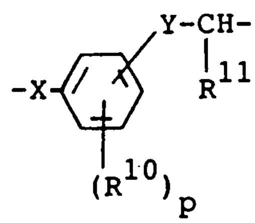

wherein $R^{10}$ and $R^{11}$ are, the same or different, each a hydrogen atom, a halogen atom or a $C_1$-$C_3$ alkyl group, X is an oxygen atom, a sulfur atom, a methylene group, a carbonyl group, a sulfoxide group, a sulfonyl group or a single bond, Y is an oxygen atom, a sulfur atom or a methylene group and p is an integer of 1 to 4;

$\ell$ is an integer of 0 to 2; and

m is an integer of 1 to 3.

2. The compound according to claim 1, wherein $R^1$ is a group of the formula:

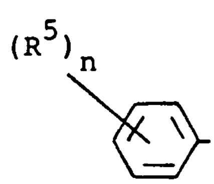

wherein $R^5$ is a hydrogen atom, a halogen atom or a $C_1$-$C_4$ alkyl group and n is an integer of 1 to 5 and A is a group

of the formula:

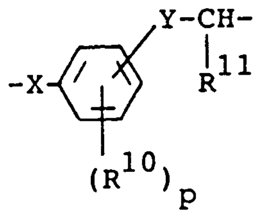

wherein $R^{10}$ and $R^{11}$ are, the same or different, each a hydrogen atom, a halogen atom or a $C_1$-$C_3$ alkyl group, p is an integer of 1 to 4, X is an oxygen atom or a methylene group and Y is an oxygen atom substituted at the p-position in regard to X and ℓ is an integer of 0 or 1.

**3.** The compound which has the formula:

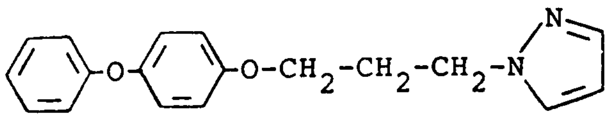

.

**4.** The compound which has the formula:

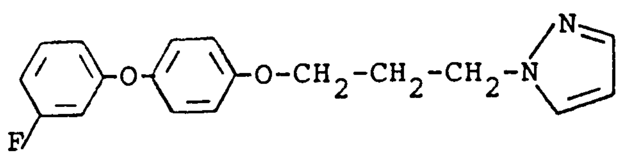

.

**5.** The compound which has the formula:

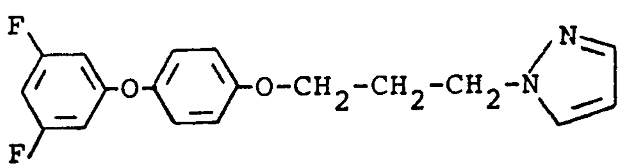

.

**6.** The compound which has the formula:

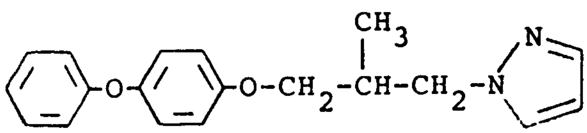

.

**7.** The compound which has the formula:

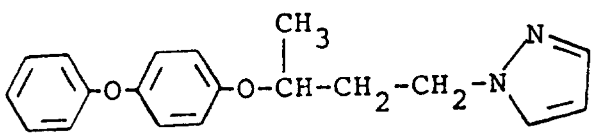

.

**8.** The compound which has the formula:

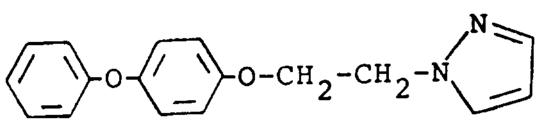

.

**9.** The compound which has the formula:

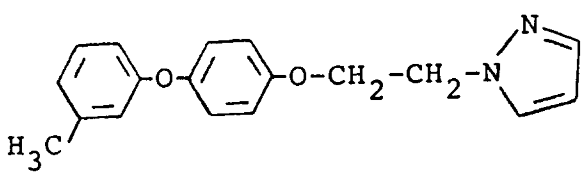

.

**10.** The compound, which has the formula:

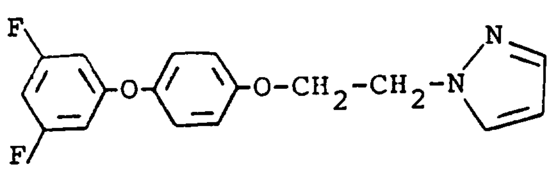

.

**11.** The compound which has the formula:

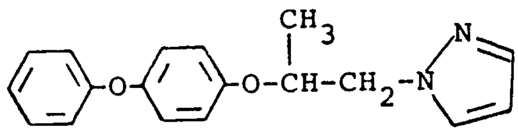

.

**12.** A process for preparing a pyrazole compound of the formula:

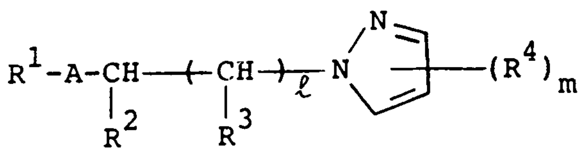

wherein

$R^1$ is a group of the formula:

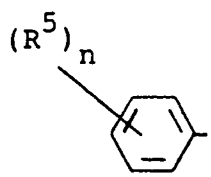

wherein $R^5$ is a hydrogen atom, a halogen atom, a hydroxy group, a cyano group, a nitro group, a $C_1$-$C_4$ alkyl group, a halo($C_1$-$C_4$)alkyl group, a $C_1$-$C_4$ alkoxy group, a halo($C_1$-$C_4$)alkoxy group, a $C_1$-$C_4$ alkylthio group, a halo($C_1$-$C_4$)alkylthio group, a $C_2$-$C_4$ alkenyl group, a $C_2$-$C_4$ alkynyl group, a $C_2$-$C_4$ alkenyloxy group, a $C_2$-$C_4$ alkynyloxy group, a $C_2$-$C_4$ alkenylthio group, a $C_2$-$C_4$ alkynylthio group, a halo($C_2$-$C_4$)alkenyl group, a halo($C_2$-$C_4$)alkynyl group, a halo($C_2$-$C_4$)alkenyloxy group, a halo($C_2$-$C_4$)alkynyloxy group or a halo($C_2$-$C_4$)alkenylthio group and n is an integer of 1 to 5;

$R^2$ and $R^3$ are, the same or different, each a hydrogen atom, a halogen atom or a $C_1$-$C_3$ alkyl group;

$R^4$ is a hydrogen atom, a halogen atom, a $C_1$-$C_4$ alkyl group or a halo($C_1$-$C_4$)alkyl group;

A is

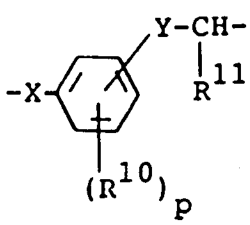

wherein $R^{10}$ and $R^{11}$ are, the same or different, each a hydrogen atom, a halogen atom or a $C_1$-$C_3$ alkyl group, X is an oxygen atom, a sulfur atom, a methylene group, a carbonyl group, a sulfoxide group, a sulfonyl group or a single bond, Y is an oxygen atom, a sulfur atom or a methylene group and p is an integer of 1 to 4;

$\ell$ is an integer of 0 to 2; and

m is an integer of 1 to 3, which comprises reacting a compound of the formula:

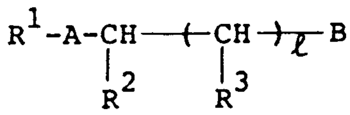

wherein $R^1$, $R^2$, $R^3$, A and $\ell$ are each as defined above and B is a halogen atom, a mesyloxy group or a tosyloxy group with a compound of the formula:

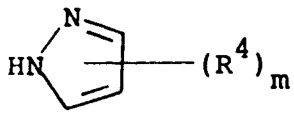

wherein $R^4$ and m are each as defined above.

**13.** A pesticidal composition which comprises as an active ingredient the pyrazole compound according to any one of claims 1 to 11 and an inert carrier or diluent.

**14.** A method for controling pests which comprises applying a pesticidally effective amount of the pyrazole compound according to any one of claims 1 to 11 to pests.

**15.** Use of the pyrazole compound according to any one of claims 1 to 11 as a pesticide.

**Patentansprüche**

**1.** Pyrazolverbindung der Formel:

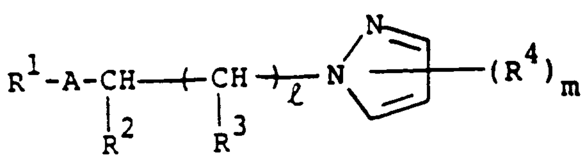

in der
$R^1$ ein Rest der Formel:

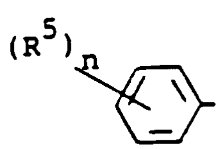

ist, in der $R^5$ ein Wasserstoffatom, ein Halogenatom, eine Hydroxylgruppe, eine Cyanogruppe, eine Nitrogruppe, ein $C_1$-$C_4$-Alkyl-, Halogen-($C_1$-$C_4$)-alkyl-, $C_1$-$C_4$-Alkoxy-, Halogen-($C_1$-$C_4$)-alkoxy-, $C_1$-$C_4$-Alkylthio-, Halogen-($C_1$-$C_4$)-alkylthio-, $C_2$-$C_4$-Alkenyl-, $C_2$-$C_4$-Alkinyl-, $C_2$-$C_4$-Alkenyloxy-, $C_2$-$C_4$-Alkinyloxy-, $C_2$-$C_4$-Alkenylthio-, $C_2$-$C_4$-Alkinylthio-, Halogen-($C_2$-$C_4$)-alkenyl-, Halogen-($C_2$-$C_4$)-alkinyl-, Halogen-($C_2$-$C_4$)-alkenyloxy-, Halogen-($C_2$-$C_4$)-alkinyloxy- oder Halogen-($C_2$-$C_4$)-alkenylthiorest ist und n eine ganze Zahl von 1 bis 5 ist,
$R^2$ und $R^3$ gleich oder verschieden sind und jeweils ein Wasserstoffatom, ein Halogenatom oder einen $C_1$-$C_3$-Alkylrest darstellen,
$R^4$ ein Wasserstoffatom, ein Halogenatom, ein $C_1$-$C_4$-Alkylrest oder ein Halogen-($C_1$-$C_4$)-alkylrest ist,

A

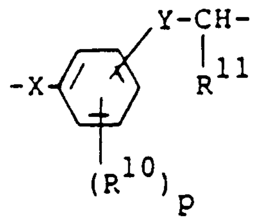

ist,
wobei $R^{10}$ und $R^{11}$ gleich oder verschieden sind und jeweils ein Wasserstoffatom, ein Halogenatom oder einen $C_1$-$C_3$-Alkylrest darstellen, X ein Sauerstoffatom, ein Schwefelatom, eine Methylengruppe, eine Carbonylgruppe, eine Sulfoxidgruppe, eine Sulfonylgruppe oder eine Einfachbindung ist, Y ein Sauerstoffatom, ein Schwefelatom oder eine Methylengruppe ist und p eine ganze Zahl von 1 bis 4 ist,
l eine ganze Zahl von 0 bis 2 ist, und
m eine ganze Zahl von 1 bis 3 ist.

**2.** Verbindung nach Anspruch 1, in der $R^1$ ein Rest der Formel:

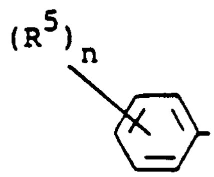

ist, in der $R^5$ ein Wasserstoffatom, ein Halogenatom oder ein $C_1$-$C_4$-Alkylrest ist und n eine ganze Zahl von 1 bis 5 ist und A ein Rest der Formel:

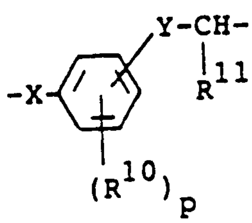

ist, in der $R^{10}$ und $R^{11}$ gleich oder verschieden sind und jeweils ein Wasserstoffatom, ein Halogenatom oder einen $C_1$-$C_3$-Alkylrest darstellen, p eine ganze Zahl von 1 bis 4 ist, X ein Sauerstoffatom oder eine Methylengruppe ist und Y ein Sauerstoffatom, substitiert in p-Stellung in bezug auf X, ist, und l eine ganze Zahl von 0 oder 1 ist.

**3.** Verbindung, die die Formel

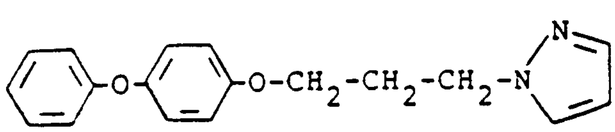

aufweist.

**4.** Verbindung, die die Formel

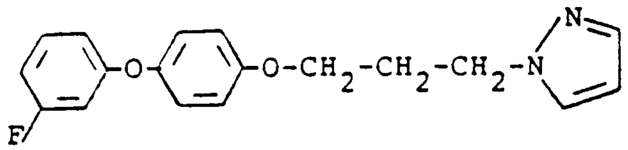

aufweist.

**5.** Verbindung, die die Formel

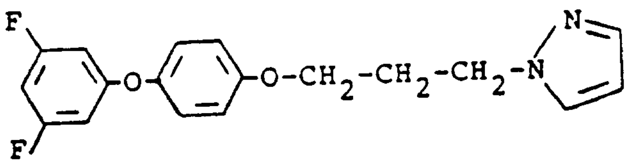

aufweist.

**6.** Verbindung, die die Formel

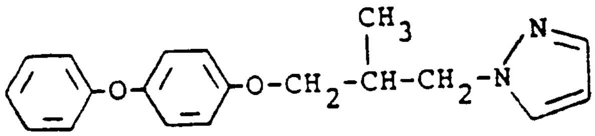

aufweist.

**7.** Verbindung, die die Formel

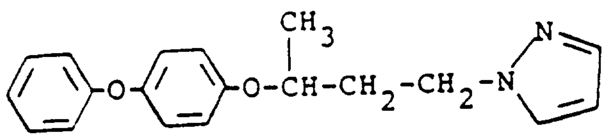

aufweist.

**8.** Verbindung, die die Formel

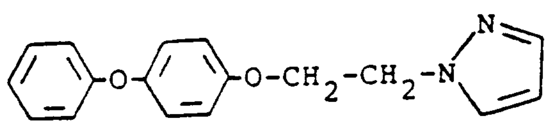

aufweist.

9. Verbindung, die die Formel

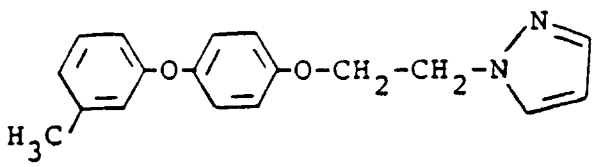

aufweist.

10. Verbindung, die die Formel

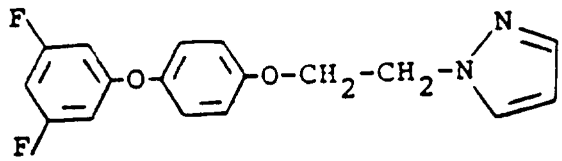

aufweist.

11. Verbindung, die die Formel

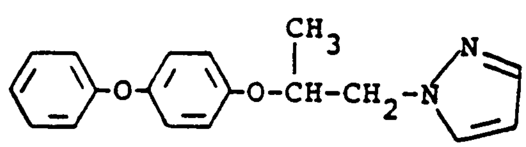

aufweist.

12. Verfahren zur Herstellung einer Pyrazolverbindung der Formel:

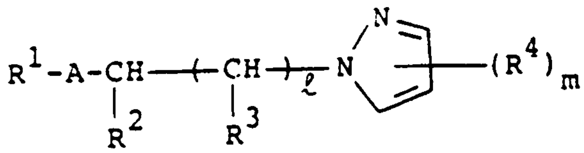

in der
$R^1$ ein Rest der Formel:

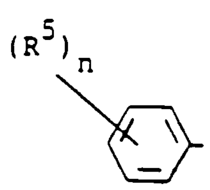

ist, in der $R^5$ ein Wasserstoffatom, ein Halogenatom, eine Hydroxylgruppe, eine Cyanogruppe, eine Nitrogruppe, ein $C_1$-$C_4$-Alkyl-, Halogen-($C_1$-$C_4$)-alkyl-, $C_1$-$C_4$-Alkoxy-, Halogen-($C_1$-$C_4$)-alkoxy-, $C_1$-$C_4$-Alkylthio-, Halogen-($C_1$-$C_4$)-alkylthio-, $C_2$-$C_4$-Alkenyl-, $C_2$-$C_4$-Alkinyl-, $C_2$-$C_4$-Alkenyloxy-, $C_2$-$C_4$-Alkinyloxy-, $C_2$-$C_4$-Alkenylthio-, $C_2$-$C_4$-Alkinylthio-, Halogen-($C_2$-$C_4$)-alkenyl-, Halogen-($C_2$-$C_4$)-alkinyl-, Halogen-($C_2$-$C_4$)-alkenyloxy-, Halogen-($C_2$-$C_4$)-alkiny-

loxy- oder Halogen-($C_2$-$C_4$)-alkenylthiorest ist und n eine ganze Zahl von 1 bis 5 ist,
$R^2$ und $R^3$ gleich oder verschieden sind und jeweils ein Wasserstoffatom, ein Halogenatom oder einen $C_1$-$C_3$-Alkylrest darstellen.
$R^4$ ein Wasserstoffatom, ein Halogenatom, ein $C_1$-$C_4$-Alkylrest oder ein Halogen-($C_1$-$C_4$)-alkylrest ist,
A

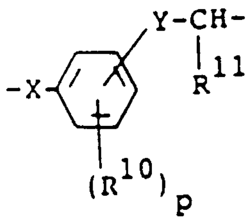

ist,
wobei $R^{10}$ und $R^{11}$ gleich oder verschieden sind und jeweils ein Wasserstoffatom, ein Halogenatom oder einen $C_1$-$C_3$-Alkylrest darstellen, X ein Sauerstoffatom, ein Schwefelatom, eine Methylengruppe, eine Carbonylgruppe, eine Sulfoxidgruppe, eine Sulfonylgruppe oder eine Einfachbindung ist, Y ein Sauerstoffatom, ein Schwefelatom oder eine Methylengruppe ist und p eine ganze Zahl von 1 bis 4 ist,
l eine ganze Zahl von 0 bis 2 ist, und
m eine ganze Zahl von 1 bis 3 ist, umfassend die Umsetzung einer Verbindung der Formel:

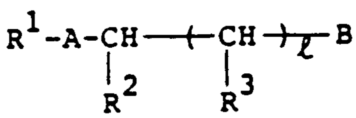

in der $R^1$, $R^2$, $R^3$, A und l jeweils die vorstehend angegebene Bedeutung haben und B ein Halogenatom, eine Mesyloxygruppe oder eine Tosyloxygruppe ist, mit einer Verbindung der Formel:

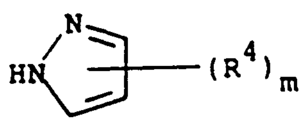

in der $R^4$ und M jeweils die vorstehend angegebene Bedeutung haben.

**13.** Pestizides Mittel, umfassend als Wirkstoff die Pyrazolverbindung nach einem der Ansprüche 1 bis 11 und einen inerten Träger oder ein Verdünnungsmittel.

**14.** Verfahren zur Bekämpfung von Schädlingen, umfassend die Anwendung einer pestizid wirksamen Menge der Pyrazolverbindung nach einem der Ansprüche 1 bis 11 auf die Schädlinge.

**15.** Verwendung der Pyrazolverbindung nach einem der Ansprüche 1 bis 11 als Pestizid.

## Revendications

1. Composé pyrazole de formule :

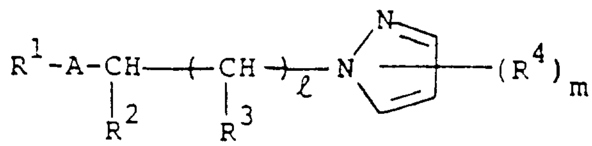

dans laquelle

$R^1$ représente un groupement de formule :

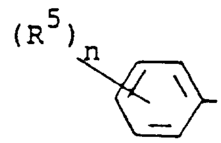

dans laquelle $R^5$ représente un atome d'hydrogène, un atome d'halogène, un groupement hydroxyle, un groupement cyano, un groupement nitro, un groupement alkyle en $C_1$-$C_4$, un groupement halogénoalkyle en $C_1$-$C_4$, un groupement alcoxy en $C_1$-$C_4$, un groupement halogénoalcoxy en $C_1$-$C_4$, un groupement alkylthio en $C_1$-$C_4$, un groupement halogéno(alkyle en $C_1$-$C_4$)thio, un groupement alcényle en $C_2$-$C_4$, un groupement alcynyle en $C_2$-$C_4$, un groupement alcényloxy en $C_2$-$C_4$, un groupement alcynyloxy en $C_2$-$C_4$, un groupement alcénylthio en $C_2$-$C_4$, un groupement alcynylthio en $C_2$-$C_4$, un groupement halogénoalcényle en $C_2$-$C_4$, un groupement halogénoalcynyle en $C_2$-$C_4$, un groupement halogéno(alcényle en $C_2$-$C_4$)oxy, un groupement halogéno(alcynyle en $C_2$-$C_4$)oxy ou un groupement halogéno(alcényle en $C_2$-$C_4$)thio et n représente un nombre entier compris entre 1 et 5;

$R^2$ et $R^3$, identiques ou différents, représentent chacun un atome d'hydrogène, un atome d'halogène ou un groupement alkyle en $C_1$-$C_3$;

$R^4$ représente un atome d'hydrogène, un atome d'halogène, un groupement alkyle en $C_1$-$C_4$ ou un groupement halogénoalkyle en $C_1$-$C_4$;

A représente

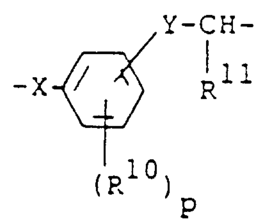

dans laquelle $R^{10}$ et $R^{11}$, identiques ou différents, représentent chacun un atome d'hydrogène, un atome d'halogène ou un groupement alkyle en $C_1$-$C_3$, X représente un atome d'oxygène, un atome de soufre, un groupement méthylène, un groupement carbonyle, un groupement sulfoxyde, un groupement sulfonyle ou une liaison simple, Y représente un atome d'oxygène, un atome de soufre ou un groupement méthylène et p représente un nombre entier compris entre 1 et 4;

$\ell$ représente un nombre entier compris entre 0 et 2; et

m représente un nombre entier compris entre 1 et 3.

**2.** Composé selon la revendication 1, pour lequel $R^1$ représente un groupement de formule :

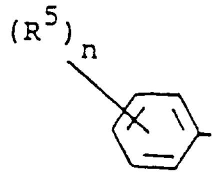

dans laquelle $R^5$ représente un atome d'hydrogène, un atome d'halogène ou un groupement alkyle en $C_1$-$C_4$ et n représente un nombre entier compris entre 1 et 5, et A représente un groupement de formule :

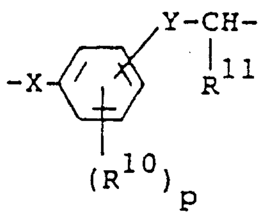

dans laquelle $R^{10}$ et $R^{11}$, identiques ou différents, représentent chacun un atome d'hydrogène, un atome d'halogène ou un groupement alkyle en $C_1$-$C_3$, p représente un nombre entier compris entre 1 et 4, X représente un atome d'oxygène ou un groupement méthylène et Y représente un atome d'oxygène substitué en position p par rapport à X et $\ell$ représente le nombre entier 0 ou 1.

**3.** Composé qui possède la formule :

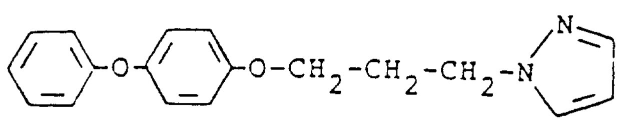

**4.** Composé qui possède la formule :

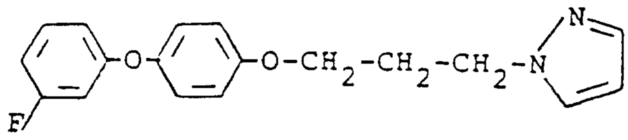

**5.** Composé qui possède la formule :

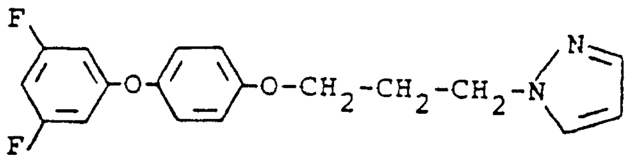

**6.** Composé qui possède la formule :

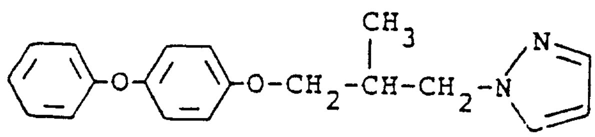

**7.** Composé qui possède la formule :

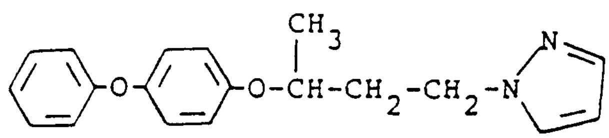

**8.** Composé qui possède la formule :

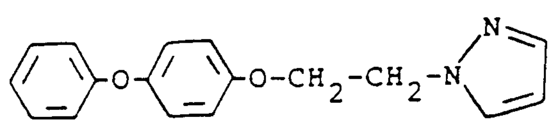

**9.** Composé qui possède la formule :

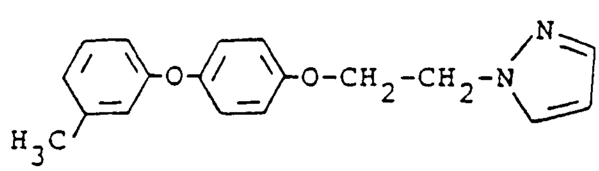

**10.** Composé qui possède la formule :

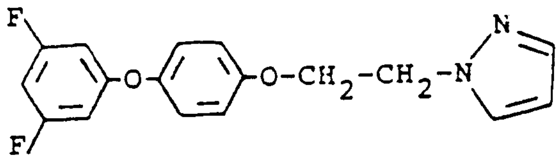

**11.** Composé qui possède la formule :

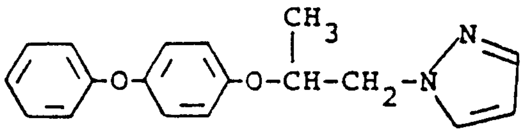

**12.** Procédé de préparation d'un composé pyrazole de formule :

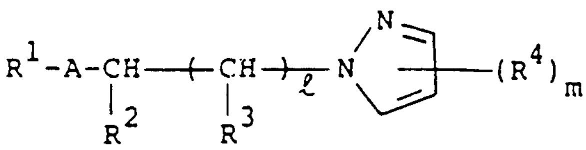

dans laquelle

$R^1$ représente un groupement de formule :

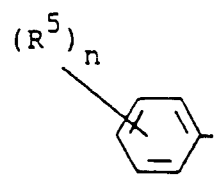

dans laquelle $R^5$ représente un atome d'hydrogène, un atome d'halogène, un groupement hydroxyle, un groupement cyano, un groupement nitro, un groupement alkyle en $C_1$-$C_4$, un groupement halogénoalkyle en $C_1$-$C_4$, un groupement alcoxy en $C_1$-$C_4$, un groupement halogénoalcoxy en $C_1$-$C_4$, un groupement alkylthio en $C_1$-$C_4$, un groupement halogéno(alkyle en $C_1$-$C_4$)thio, un groupement alcényle en $C_2$-$C_4$, un groupement alcynyle en $C_2$-$C_4$, un groupement alcényloxy en $C_2$-$C_4$, un groupement alcynyloxy en $C_2$-$C_4$, un groupement alcénylthio en $C_2$-$C_4$, un groupement alcynylthio en $C_2$-$C_4$, un groupement halogénoalcényle en $C_2$-$C_4$, un groupement halogénoalcynyle en $C_2$-$C_4$, un groupement halogéno(alcényle en $C_2$-$C_4$)oxy, un groupement halogéno(alcynyle en $C_2$-$C_4$)oxy ou un groupement halogéno(alcényle en $C_2$-$C_4$)thio et n représente un nombre entier compris entre 1 et 5;

$R^2$ et $R^3$, identiques ou différents, représentent chacun un atome d'hydrogène, un atome d'halogène ou un groupement alkyle en $C_1$-$C_3$;

$R^4$ représente un atome d'hydrogène, un atome d'halogène, un groupement alkyle en $C_1$-$C_4$ ou un groupement halogénoalkyle en $C_1$-$C_4$;

A représente

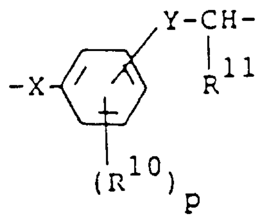

dans laquelle $R^{10}$ et $R^{11}$, identiques ou différents, représentent chacun un atome d'hydrogène, un atome d'halogène ou un groupement alkyle en $C_1$-$C_3$, X représente un atome d'oxygène, un atome de soufre, un groupement méthylène, un groupement carbonyle, un groupement sulfoxyde, un groupement sulfonyle ou une liaison simple, Y représente un atome d'oxygène, un atome de soufre ou un groupement méthylène et p représente un nombre entier compris entre 1 et 4;

$\ell$ représente un nombre entier compris entre 0 et 2; et

m représente un nombre entier compris entre 1 et 3, lequel comprend la réaction d'un composé de formule :

$$R^1\text{-}A\text{-}\underset{R^2}{CH}\text{-}(\text{-}\underset{R^3}{CH}\text{-})_\ell\text{-}B \qquad \text{(II)}$$

dans laquelle $R^1$, $R^2$, $R^3$, A et $\ell$ sont tels que définis ci-dessus et B représente un atome d'halogène, un groupement mésyloxy ou un groupement tosyloxy, avec un composé de formule :

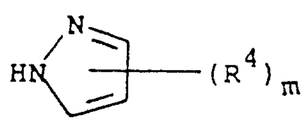

dans laquelle $R^4$ et m sont tels que définis ci-dessus.

**13.** Composition pesticide qui comprend, comme principe actif, le composé pyrazole selon l'une quelconque des revendications 1 à 11 et un support ou diluant inerte.

**14.** Procédé de lutte contre les insectes nuisibles qui comprend l'application d'une quantité active comme pesticide du composé pyrazole selon l'une quelconque des revendications 1 à 11 aux insectes nuisibles.

**15.** Utilisation du composé pyrazole selon l'une quelconque des revendications 1 à 11 comme pesticide.